# EUROPEAN PATENT SPECIFICATION

(11) **EP 2 504 050 B1**
(45) Date of publication and mention of the grant of the patent: **04.09.2019**
(21) Application number: 10779554.4
(22) Date of filing: 22.11.2010
(51) Int. Cl.: A61M 15/00

(54) **NEBULIZER**
ZERSTÄUBER
NÉBULISEUR

(30) Priority: 25.11.2009 EP 09014679
(43) Date of publication of application: 03.10.2012
(73) Proprietor: Boehringer Ingelheim International GmbH, 55216 Ingelheim am Rhein (DE)
(72) Inventor: BACH, Alexander, 55216 Ingelheim Am Rhein (DE); BESSELER, Jens, 55216 Ingelheim Am Rhein (DE); GOLBERG, Christian, 55216 Ingelheim Am Rhein (DE); HERRMANN, Frank, 55216 Ingelheim Am Rhein (DE); HOLAKOVSKY, Holger, 55216 Ingelheim Am Rhein (DE); DAELMAN, Manuel, 55216 Ingelheim Am Rhein (DE); THOEMMES, Ralf, 55216 Ingelheim Am Rhein (DE); WUTTKE, Gilbert, 55216 Ingelheim Am Rhein (DE)
(74) Representative: Simon, Elke Anna Maria
(86) International application number: PCT/EP2010/067896
(87) International publication number: WO 2011/064160

(56) References cited:
- WO-A1-95/32015
- WO-A2-2006/125577
- WO-A2-2008/138936
- FR-A1- 2 604 363
- US-A- 4 178 928
- US-A1- 2005 131 357
- US-A1- 2007 029 475
- US-B2- 6 772 915

## Description

The present invention relates to a nebulizer according to the preamble of claim 1.

The starting point for the present invention is a nebulizer illustrated in WO 2006/125577 A2. The nebulizer has, as a reservoir for fluid which is to be atomized, an insertable rigid container having an inner bag containing the fluid and a pressure generator with a drive spring for delivering and atomizing the fluid.

Preferably, the container is pre-installed in nebulizer in the delivery state. Before being used for the first time the nebulizer is completely closed. Thus, the pre-installed container is opened by a delivery tube piercing a sealing and a septum to fluidically connect to the inner bag of the container.

By rotating the lower housing part of the nebulizer the drive spring can be put under tension and fluid can be sucked into a compression chamber of the pressure generator. Simultaneously, the container is moved into the lower housing part in a stroke movement within the nebulizer and when tensioned for the first time the container may be pierced through its base by a piercing element in the lower housing part to allow venting of the container. After manual operation of a locking element the drive spring is released and the fluid in the pressure chamber is put under pressure by the drive spring and is delivered or atomized through a nozzle into a mouthpiece as an aerosol, without the use of propellant gas.

WO2008/138936 A2 discloses an atomizer which is similar to the nebulizer of WO 2006/125577 A2 and which additionally comprises a protective device for preventing germs from infesting the fluid. A delivery condition for the atomizer is disclosed wherein the atomizer with a pre-installed, closed container is surrounded by a pouch.

US 2007/029475 A1 discloses another related nebulizer with a spring-driven pressure generator for delivering and atomizing the fluid from a container.

The nebulizer comprises a conveying tube for conveying the fluid from the container into the pressure generator, wherein the conveying tube is of a multipart construction by which a double-wall is formed.

US 6772915 B2 discloses a dispenser for discharging a liquid containing a pharmaceutical substance in a container wherein the dispenser is constructed in such a way that the container with the storage chamber can be introduced already at the pharmaceutical filling company. The container comprises a storage chamber which is constructed as a pump chamber of a thrust piston pump. The thrust piston is constructed as a plug sealing the pump chamber. Serving as a piston rod, there is a discharge channel leading to a discharge opening in the housing. As a result of the actuation of an actuating means (held on the housing by means of an elastically deformable material bridge), a relative movement is produced between the actuating means and a housing.

During a first actuation, as soon as the actuating force on the actuating means is high enough for counteracting locking actions of detents, a needle point of the piston rod perforates the plug (i.e. the connection between pump chamber and discharge opening is formed). Then, during the further actuation of the dispenser, the piston is moved in such a way that the pump chamber volume is reduced and as a result the medium is discharged through the discharge opening.

Other prior art devices are disclosed in WO95/32015 A1 and US2005/131357 A1.

Object of the present invention is to provide a nebulizer with minimized size even with pre-installed container.

The above object is achieved by a nebulizer according to claim 1. Preferred embodiments are subject of the subclaims.

A basic idea of the present invention is that even in its delivered state the nebulizer has a closed container provided therein and the nebulizer is constructed so that the container is opened inside the nebulizer before or during the first use of the nebulizer. This basic idea is hereinafter called also "pre-installed container". This makes operation easier as there is no need to open the nebulizer, insert the container and close the nebulizer. Moreover, undesirable soiling or damage to the nebulizer caused by incorrect handling when inserting the container can thus be prevented. Accordingly, there is better operational safety as it is impossible for the container to be wrongly inserted or otherwise misused during insertion.

According to a first aspect of the present invention, the nebulizer comprises a conveying element for fluidically connecting the container. In the delivery state, the conveying element is already partly inserted into the container, but a first closure closing the fluid outlet of the container is still closed. This closure is opened during further insertion of the conveying element. Thus, it is possible to minimize the size of the nebulizer with the pre-installed container in the delivery state.

According to a second aspect of the present invention, the container comprises a first closure and a second closure which are both associated to a fluid outlet of the container for closing the fluid outlet. In the delivery state, the second or outer closure is already opened by the nebulizer in a first step, in particular when inserting the container into the nebulizer and/or when - at least partially - closing the nebulizer to pre-install the container. However, the first closure is still closed in this state. The first closure is opened later during a separate second step inside the nebulizer before or during the first use of the nebulizer. Thus, it is possible to minimize the size of the nebulizer with the pre-installed container in the delivery state.

In particular, the present invention allows that a (lower) housing part of the nebulizer can be pushed into or onto the nebulizer or its housing further than in case of the prior art, so that the second closure is already opened, in particular pierced, in the delivery state, but the first closure is still closed. Consequently, the nebulizer is smaller, in particular in axial direction, in the delivery state than the nebulizer according to the prior art.

Preferably, the container is not replaceable and in particular cannot be removed. This again leads to easier operation and hence improved operational reliability. This also prevents the nebulizer from being used or re-used in an undesirable or unauthorized manner.

In particular, the nebulizer cannot be opened and a lower housing part cannot be removed in order to replace the empty container with a full one in an undesirable manner.

The combination of the pre-installed container and the construction which makes the container non-replaceable results in particularly easy operation and high operational reliability as the user can only use the nebulizer as a single-use item until the container is empty, and undesirable or unauthorized further use of the nebulizer is prevented by the fact that the container cannot be replaced.

However, correspondingly easy operation and improved operational reliability for the user can also be achieved if the container is pre-installed at the pharmacy, for example, i.e. by trained staff, and optionally opened at the same time provided that the container is made non-exchangeable, in particular the nebulizer cannot be opened by the user.

Further advantages, features, characteristics and aspects of the present invention will become apparent from the claims and the following description of a preferred embodiment with reference to the drawings. It shows:
- Fig. 1: a schematic section of a known nebulizer in a non-tensioned state;
- Fig. 2: a schematic section, rotated through 90° compared with Fig. 1, of the known nebulizer in a tensioned state;
- Fig. 3: a schematic section of a nebulizer according to the present invention in a delivery state with a partly closed housing and with a pre-installed, closed container;
- Fig. 4: a schematic section of the nebulizer according to Fig. 3 in an activated or tensioned state with the completely closed housing and with the opened container; and
- Fig. 5: a schematic section of the nebulizer according to Fig. 4 in a non-tensioned state.

In the Figures, the same reference numerals have been used for identical or similar parts, resulting in corresponding or comparable properties and advantages, even if the associated description is not repeated.

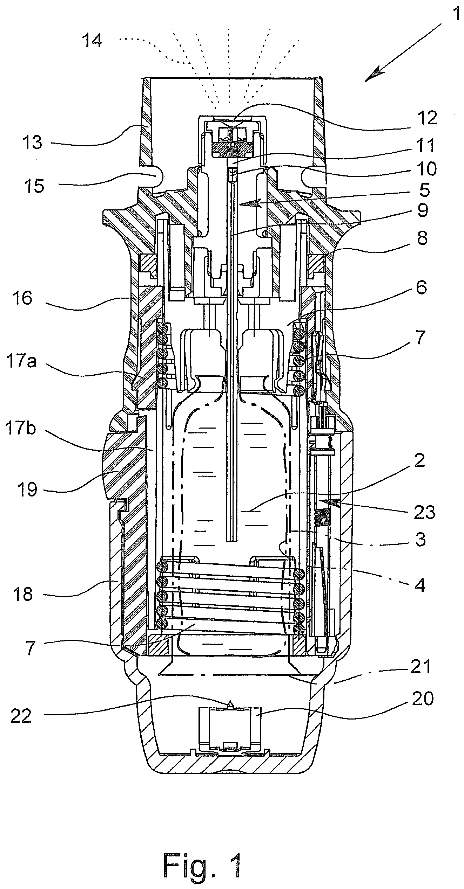

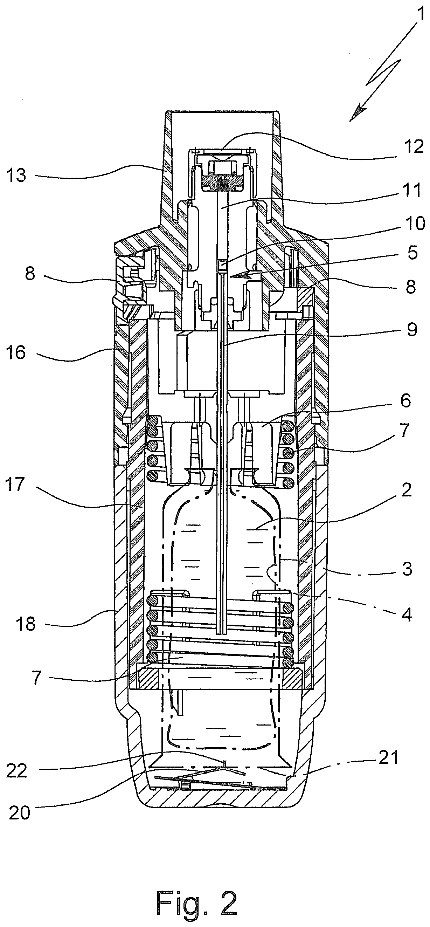

Figs. 1 and 2 show a known nebulizer 1 for atomizing a fluid 2, particularly a highly effective pharmaceutical composition or the like, diagrammatically shown in a non-tensioned state (Fig. 1) and in a tensioned state (Fig. 2). The nebulizer 1 is constructed in particular as a portable inhaler and preferably operates only mechanical and/or without propellant gas.

When the fluid 2, preferably a liquid, more particularly a pharmaceutical composition, is nebulized, an aerosol 14 (Fig. 1) is formed, which can be breathed in or inhaled by a user. Usually the inhaling is done at least once a day, more particularly several times a day, preferably at set intervals, depending on the complain or illness from which the patient is suffering.

The nebulizer 1 is provided with or comprises an insertable container 3 containing the fluid 2. The container 3 thus forms a reservoir for the fluid 2 which is to be nebulized. Preferably, the container 3 contains an amount of fluid 2 or active substance which is sufficient to provide up to 200 dosage units, for example, i.e. to allow up to 200 sprays or applications. A typical container 3, as disclosed in WO 96/06011 A1, holds e.g. a volume of about 2 to 10 ml.

The container 3 is substantially cylindrical or cartridge-shaped and once the nebulizer 1 has been opened the container can be inserted therein from below and changed if desired. It is preferably of rigid construction, the fluid 2 in particular being held in a collapsible bag 4 in the container 3.

The nebulizer 1 comprises preferably a pressure generator 5 for conveying and nebulizing the fluid 2, particularly in a preset and optionally adjustable dosage amount. The pressure generator 5 comprises preferably a holder 6 for the container 3, an associated drive spring 7, only partly shown, a locking element 8 which can be manually operated to release the spring 7, a conveying element, such as a conveying tube 9, a non-return valve 10, a pressure chamber 11 and/or an nozzle 12 for nebulizing the fluid 2 into a mouthpiece 13. The container 3 is fixed or held in the nebulizer 1 via the holder 6 such that the conveying tube 9 penetrates into the container 3. The holder 6 may be constructed so that the container 3 can be exchanged.

As the drive spring 7 is axially tensioned the holder 6 with the container 3 and the conveying tube 9 is moved downwards in the drawings and fluid 2 is sucked out of the container 3 into the pressure chamber 11 of the pressure generator 5 through the non-return valve 10. Then, the nebulizer 1 is in the so called activated or tensioned state.

During the subsequent relaxation after actuation of the locking element 8 the fluid 2 in the pressure chamber 11 is put under pressure as the conveying tube 9 with its now closed non-return valve 10 is moved back upwards by the relaxation of the drive spring 7 and now acts as a pressing ram or piston. This pressure forces the fluid 2 through the nozzle 12, whereupon it is nebulized into the aerosol 14, as shown in Fig. 1. The preferred droplet size of the particles has already been discussed above in the introductory part.

Generally, the nebulizer 1 operates with a spring pressure of 5 to 200 MPa, preferably 10 to 100 MPa on the fluid 2, with a volume of fluid 2 delivered per stroke of 10 to 50 µl, preferably 10 to 20 µl, most preferably about 15 µl. The fluid 2 is converted into or nebulized as aerosol 14 the droplets of which have an aerodynamic diameter of up to 20 µm, preferably 3 to 10 µm. Preferably, the generated jet spray has an angle of 20° to 160°, preferably 80° to 100°. These values also apply to the nebulizer 1 according to the teaching of the present invention as particularly preferred values.

A user (not shown) can inhale the aerosol 14, while an air supply can be sucked into the mouthpiece 13 through at least one air supply opening 15.

Preferably, the nebulizer 1 or drive spring 7 can be manually activated or tensioned. The nebulizer 1 comprises preferably an upper housing part 16 and an inner part 17 which is rotatable relative thereto (Fig. 2) having an upper part 17a and a lower part 17b (Fig. 1), while an in particular manually operable (lower) housing part 18 is releasably fixed, particularly fitted onto the inner part 17, preferably by means of a retaining element 19. Preferably, the housing parts 16 and 18 form a housing of the nebulizer 1. In order to insert and/or replace the container 3 the housing part 18 can be detached from the nebulizer 1 or its housing.

The housing part 18 can be rotated relative to the upper housing part 16, carrying with it the part 17b of the inner part 17. As a result the drive spring 7 is tensioned in the axial direction by means of a gear or transmission (not shown) acting on the holder 6. During tensioning the container 3 is moved axially downwards until the container 3 assumes an end position as shown in Fig. 2. In this activated or tensioned state the drive spring 7 is under tension. During the nebulizing process the container 3 is moved back into its original position (non-tensioned position or state shown in Fig. 1) by the drive spring 7. Thus the container 3 executes a lifting or stroke movement during the tensioning process and during the atomizing process.

The housing part 18 preferably forms a cap-like lower housing part and fits around or over a lower free end portion of the container 3. As the drive spring 7 is tensioned the container 3 moves with its end portion (further) into the housing part 18 or towards the end face thereof, while an aeration means, such as an axially acting spring 20 arranged in the housing part 18, comes in contact with base 21 of the container 3 and pierces the container 3 or a base seal thereon with a piercing element 22 when the container 3 makes contact with it for the first time, to allow air in or aeration.

The nebulizer 1 may comprise a monitoring device 23 which counts the actuations of the nebulizer 1, preferably by detecting the rotation of the inner part 17 relative to the upper part 16 of the housing. Preferably, the monitoring device 23 blocks the actuation or use of the nebulizer 1, e.g. blocks the actuation of the locking element 8, when a certain number of actuations or discharged doses has been reached or exceeded.

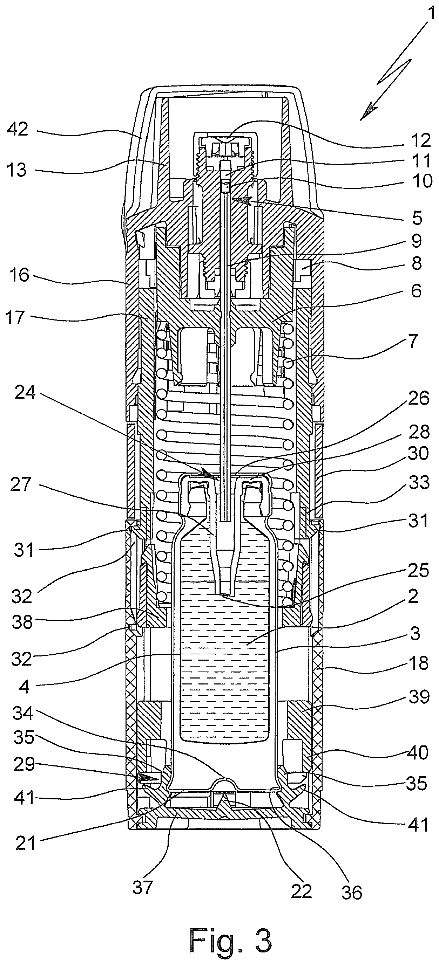

The construction and mode of operation of a proposed inhaler or nebulizer 1 will now be described in more detail with reference to Fig. 3 to 5, but emphasizing only essential differences from the nebulizer 1 according to Figs. 1 and 2. The remarks relating to Figs. 1 and 2 thus apply preferably accordingly or in a similar manner, while any desired combinations of features of the nebulizer 1 according to Figs. 1 and 2 and the nebulizer 1 described below are possible.

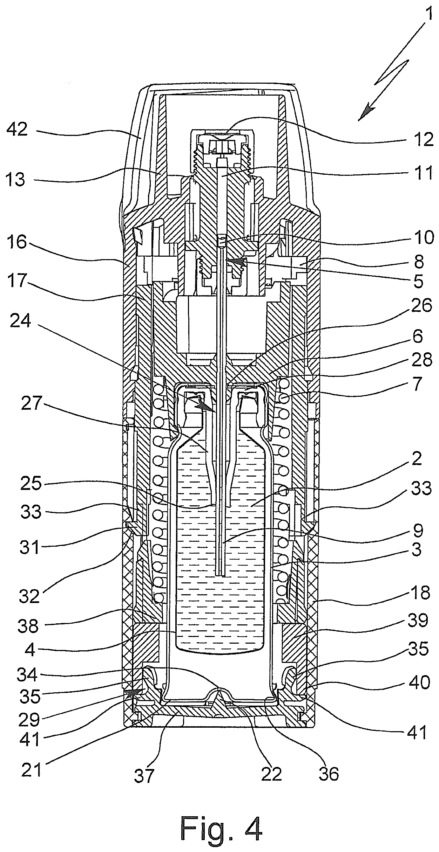

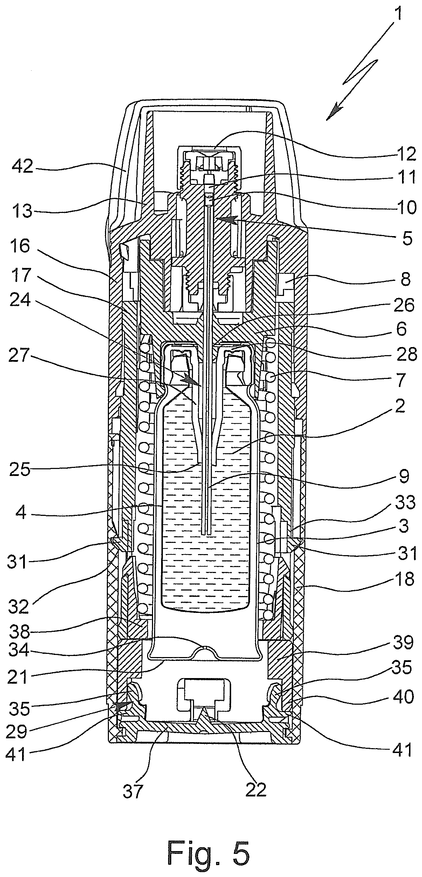

Figs. 3 to 5 show, in schematic sectional views, a nebulizer 1 according to a preferred embodiment of the present invention. Fig. 3 shows the nebulizer 1 in a delivery state, i.e. with pre-installed container 3 which is still closed. In this state, the housing of the nebulizer 1 is not completely closed, in particular the housing part 18 is not completely pushed on the inner part 17. Fig. 4 and 5 show the nebulizer 1 in an activated state with the housing completely closed and with the container 3 opened. In Fig. 4, the nebulizer 1 or drive spring 7 is tensioned, i. e. the container 3 is in its lower position. Fig. 5 shows the nebulizer 1 in a non-tensioned state, e.g. after the delivery or discharge of one dose of the fluid 2, the container 3 is in its upper position.

The container 3 is already mounted or pre-installed in the nebulizer 1 in the delivery state, as shown in Fig. 3. In this state, the container 3 is still closed, i.e. there is no fluidic connection between the container 3 or its bag 4 on one hand and the nebulizer 1 or its pressure generator 5 or the conveying element on the other hand.

The container 3 comprises a fluid outlet 24 for outputting the fluid 2 to be dispensed. In particular, the fluid outlet 24 allows a fluidic connection between the container 3 or its bag 4 on one hand and the nebulizer 1, its pressure generator 5 or the conveying element on the other hand.

In the non-installed state of the container 3, i.e. before mounting or pre-installation of the container 3 in the nebulizer 1, the fluid outlet 24 is closed by a first or inner closure 25 and optionally by a second or outer closure 26. In particular, the second closure 26 covers the first closure 25.

The first or inner closure 25 is preferably formed by a septum, a membrane, a plastic seal or the like and/or is provided inside the container 3.

In the preferred embodiment, the second closure 26 is preferably formed by a seal, a foil, a cap or the like, in particular by a metallic and/or composite foil or the like, which is preferably hot-sealed or attached in any other suitable manner on or to a head end or axial end of the container 3. In the shown embodiment, the second closure 26 is formed preferably by a hot-sealed foil with an aluminum layer.

Preferably, the closures 25 and 26 are designed such that separate opening is possible, in particular such that the second closure 26 can be opened independently from the first closure 25 and/or has to be opened before the first closure 25.

Preferably, the closures 25 and 26 are designed such that successive opening is possible by means of one common element, in particular the conveying element or conveying tube 9 or the like, and/or by piercing.

In the preferred embodiment, the first closure 25 and second closure 26 are arranged one after the other and/or spaced in axial direction or direction of the stroke movement of the container 3 or with respect to the main outlet direction of the fluid 2.

Preferably, the first or inner closure 25 is formed or supported by a closure part 27 extending from the outlet or head end of the container 3 into the container 3 or bag 4. The second or outer closure 26 is preferably located adjacent to the head or axial end of the container 3 and/or held or connected to a flange 28, which can be formed by the closure part 27 or any other suitable part. However, other constructional solutions are possible.

In the delivery state according to Fig. 3, the container 3 has been pre-installed, i.e. inserted into the nebulizer 1. However, the container 3 or its fluid outlet 24 is not yet opened. In particular, the second closure 26 is already opened, but not the first closure 25. This is achieved in particular in that the housing of the nebulizer 1 is closed only partly, i.e. not completely, in the delivery state, preferably by not completely closing or pushing on the housing part 18 in the shown embodiment. Preferably, the housing part 18 is snapped on or inserted only partly in the delivery state.

Generally, the container 3, fluid outlet 24 or closures 25 or 26 are opened in particular by means of a conveying element, such as the conveying tube 9, or the like and/or by piercing or in any other suitable manner. In particular, the opening is achieved by moving the container 3 relative to the nebulizer 1 or conveying element or tube 9 or the like and/or by movement in longitudinal or axial direction.

According to the present invention, the second closure 26 is already opened in the delivery state, preferably automatically by the nebulizer 1. In particular, the second closure 26 is opened during or by or when inserting the container 3 and/or during, by or when - preferably partly - closing the housing or housing part 18 of the nebulizer 1. Preferably, the first closure 25 is designed such that, when the conveying element pierces or opens the first closure 25, such as a septum, any material may not fall into the fluid 2, but will stay connected to the closure part 27 or the like and/or will be pivoted aside.

In particular, the container 3 is attached to or held by or secured in the housing part 18, in particular by a transportation lock 29, which is preferably arranged within or at the housing part 18. The transportation lock 29 holds the container 3 preferably temporarily, in particular before attaching the housing part 18 to the nebulizer 1 and/or in the delivery state. In particular, the transportation lock 29 holds the container fixed during the fluidic connection of container 3 and/or during the mechanic of container 3, here with holder 6.

Preferably, the second closure 26 is automatically opened, in particular pierced, when pre-installing the container 3 and/or attaching the housing part 18 to the nebulizer 1, in particular when snapping or pushing the housing part 18 partly on the nebulizer 1. Then, the opening or piercing is effected in the preferred embodiment by the conveying element or conveying tube 9 which extends in the delivery state through the second closure 26 and in particular into the closure part 27, i.e. partly into the container 3. Thus, a very compact arrangement and a small size or axial extension of the nebulizer 1 can be achieved in the delivery state. In particular, the housing part 18 can be snapped or pushed on or inserted into the nebulizer 1 or its housing in the delivery state significantly further than in case of the prior art.

In the delivery state, the first closure 25 and, thus the container 3 and the fluid outlet 24 remain closed.

In the delivery state, the nebulizer 1 or the housing part 18 is preferably secured, in particular by means of a securing means or member 30, such that the container 3 and/or housing part 18 are held sufficiently spaced from the nebulizer 1 or upper housing part 16 and/or prevented from being completely inserted or pushed on the conveying element or tube 9, the housing or inner housing part 17 or the like and/or such that (complete) opening of the container 3, namely of the first closure 25, is prevented.

In the shown embodiment, the securing means or member 30 is preferably mounted between the housing part 18 and the upper housing part 16 and preferably engages with or between the housing parts 16 and 18, so that the housing part or lower part 18 is axially secured or is kept or held sufficiently away or spaced from the upper housing part 16 to be able to hold the (still) closed container 3 or first closure 25 away from the conveying tube 9.

In the preferred embodiment, the securing member 30 is at least substantially hollow and/or cylindrical and is disposed axially between the (lower) housing part 18 and the upper housing part 16. To activate the nebulizer 1 or prepare its for use, i.e. to push the housing part 18 fully on in the axial direction and thereby open the container 3, the securing member 30 first has to be removed or released or opened. In the shown preferred embodiment, the securing member 30 is constructed in the manner of a banderole or the like, made of plastics, for example, and/or can be manually opened, removed or destroyed. The securing member 30 may alternatively or simultaneously form or constitute a seal of origin. However, other embodiments of the securing member 30 are also possible, e.g. in the form of a security tag or the like.

Preferably, the container 3 and/or housing part 18 are held positively or in a form-fit or interlocking manner in the delivery state. This is achieved in the preferred embodiment in particular by means of the transportation lock 29 acting between the container 3 and the housing part 18, and the securing means or member 30 acting between the housing part 18 and the housing of the nebulizer 1 or the upper housing part 16 or the like. However, the transportation lock 29 or securing means or member 30 could also act directly between the container 3 on one hand and the nebulizer 1, its housing, the upper housing part 16, the inner housing part 17 or the holder 6 on the other hand.

The pre-installed container 3, i.e. its first closure 25, is still closed in the delivery state, i.e. non-activated state with pre-installed container 3. In this non-activated position, the housing part 18 is preferably secured so that it cannot be lost and, in particular, cannot be released. Then, the housing part or lower part 18 of the nebulizer 1 can no longer be detached from the nebulizer 1 after it has been (partially) axially pushed on for the first time, i.e. the nebulizer 1 cannot be opened any longer, with the result that that the container 3 cannot be changed, i.e. cannot be removed again.

In order to secure the housing part 18, it is preferably held or latched positively or in an interlocking or form-fit manner. Preferably, the housing part 18 is secured by means of at least one latching lug 31, protrusion, nose or the like which engages in an associated latching recess 32 in the housing part 18 or the like and, thereby, secures the housing part 18 against axial removal by interlocking engagement. In the present embodiment, the latching lug 31 may be formed by or at a latching arm 33 which can preferably flex. Thus, a ratchet-like means for securing the housing part 18 to the nebulizer 1 or its housing or the upper housing part 16 is formed. However, other constructional solutions are also possible.

Once the security member 30 has been removed a user (not shown) can push the housing part 18 fully on in the axial direction and thereby open the container 3, i.e. first closure 25, by inserting the conveying element or conveying tube 9. Figs. 4 and 5 show this activated state with the housing part 18 pushed fully on and/or the container 3 open (fluidically connected to the nebulizer 1 or its pressure generator 5 or the conveying element or tube 9). In this pushed on or activated state, the housing part 18 is preferably secured or axially fixed again by interlocking engagement, i.e. form-fit manner in axial direction, particularly by the engagement of the latching arm 33 or latching lug 31 in a corresponding further latching recess 32 or by means of some other mechanical securing device.

Fig. 4 shows the nebulizer 1 or container 3 in the activated state, the container 3, i.e. first closure 25, is open, i.e. the container 3 or its fluid 2 is fluidically connected to the nebulizer 1 or its pressure generator 5, and the housing part 18 has been pushed fully on in the axial direction. In order to bring the holder 6 into (complete) engagement with the container 3 at the head end and then be able to move the container 3 back and/or forth for the suction/tensioning and pressing strokes, it may be necessary to tension the nebulizer 1 or it drive spring 7 for the first time. During this tensioning process the holder 6 is moved together with the conveying tube 9 axially towards or into the housing part 18, thus bringing the holder 6 into (complete) engagement with the container 3 and preferably also moving or pressing the container 3 against the piercing element 22 in the region of the base of the housing part 18 and thereby piercing or opening a vent opening 34 in the container base 21. Fig. 4 shows the nebulizer 1 in this tensioned and activated state. The holder 6 is engaged with the container 3 and the conveying tube 9 has been fully inserted into the container 3.

Fig. 5 shows the nebulizer 1 in the relaxed, non-tensioned state, i.e. after atomization or discharge of a dose of the fluid 2. The holder 6 and the container 3 are in the upper position. The holder 6 is still engaged with the container 3 and remains engaged during the further uses of the nebulizer 1. Further, the container 3 is still open and fludically connected, i.e. the nebulizer 1 remains activated.

In the delivery state shown in Fig. 3, i.e. with the container 3, namely the first closure 25, (still) closed, the nebulizer 1 can be shipped or delivered to the user. Then, the user can store the nebulizer 1 with the pre-installed container 3. The container 3 will be opened later before or during the first use of the nebulizer 1, namely when removing the securing member 30 and completely closing the nebulizer 1 or housing or housing part 18.

It should be noted that the opening of the container 3 is preferably carried out exclusively by mechanical means and / or manual actuation. However, it is additionally or alternatively possible to open it in other ways, e.g. by chemical, electrical, magnetic, pneumatic, hydraulic or similar means.

The proposed nebulizer 1 is activated after the removal of the securing member 30 and (total) axial pushing on of the housing part 18 and can be used in the same way as the nebulizer 1 shown in Figs. 1 and 2. The pre-installation of the container 3 prevents the wrong container 3 or used containers 3 from being inserted in the nebulizer 1 by the user. Additionally it ensures that a separately supplied container 3 is not accidentally opened before being inserted in the nebulizer 1. Additionally the proposed solution prevents possible soiling or damage to the nebulizer 1, e.g. the conveying tube 9 or the like, when the nebulizer 1 is opened and the container 3 is used improperly.

As preferably the container 3 cannot then be removed, especially because the nebulizer 1 cannot be opened and the housing part 18 cannot be removed again, undesirable replacement of the container 3 by the user and in particular undesirable interim or subsequent opening of the nebulizer 1 by the user can be prevented.

To prevent unwanted opening of the container 3, particularly of the first closure 25, in the delivery state of the nebulizer 1, preferably the transportation lock 29 is provided. By frictional, forcible or interlocking engagement, for example, the transportation lock 29 prevents the container 3 from undesirably moving axially in the nebulizer 1, e.g. during transportation, in the event of accidental dropping of the nebulizer 1 or the like.

In the following, a preferred realization of the transportation lock 29 will be explained. It has to be noted that the transportation lock 29 can be realized independently from the preferred partial opening or piercing of the container 3 in the delivery state, in particular namely opening of the second closure 26. In particular, the proposed function and construction of the transportation lock 29 can be realized independently from the features of the present claims.

In the preferred embodiment, the transportation lock 29 comprises at least one gripping arm 35, preferably a plurality of gripping arms 35, for axially holding the container 3 in the delivery state, in particular by (radially) engaging around its preferably radially expanded base 21 or edge 36, as shown in Fig. 3.

The gripping arms 35 are preferably held or formed by or attached to or moulded unitary with a member 37 which may form the bottom or base or end face of the housing part 18. Preferably, the member 37 or bottom holds the gripping arms 35 such that the arms 35 can flex or pivot.

Preferably, the piercing element 22 is also formed by or held by the member 37.

It has to be noted that the member 37 and/or the transportation lock 29 may be inserted into the housing part 18. The transportation lock 29 or part thereof can also be formed by or in the housing part 18.

Preferably, the transportation lock 29 is formed by multiple or only two different parts, here the gripping arm(s) 35 and a control member 39 as explained later.

The transportation lock 29, in particular, the gripping arms 35, are holding the container 3 in the delivery state (closed transportation lock 29) preferably such that the container base 21 or venting opening 34 are axially spaced from the piercing element 22, as shown in Fig. 3.

To open the transportation lock 29, the gripping arms 35 may be flexed radially outwardly. Preferably, the opening of the transportation lock 29 or the flexing of the gripping arms 35 occurs automatically when closing the nebulizer 1 or its housing completely, i.e. when snapping or pushing on the housing part 18 completely towards the upper housing part 16. During this (axial or telescopic) closing movement, the transportation lock 29 is opened and the container 3 released in axial direction preferably only in a last part of the movement and/or just little before the final completely closed position is reached or just when the final completely closed position is reached.

The closing movement of the nebulizer 1 opens the transportation lock 29 preferably automatically. In particular, the transportation lock 29 is opened by the direct or indirect interaction with or actuation by the housing of the nebulizer 1, the inner part 17 or its lower part 17b, a holding ring 38 bearing the spring 7 or the like. Preferably, the container 3 and/or first closure 25 are opened as well as the transportation lock 29 by means of a common actuation, here the closing movement of the nebulizer 1 or its housing or bottom part 18.

In the preferred embodiment, the transportation lock 29 comprises a control member 39, in particular a ring or the like, for actuating or opening or engaging with or pivoting preferably all gripping arms 35 simultaneously. In particular, the control member 39 or transportation lock 29 may convert a linear or axial movement into a pivot or radial movement of the gripping arms 35.

The control member 39 is shown in an upper position in Fig. 3 when the transportation lock 29 is closed. In this position, the control member 39 may secure the gripping arms 35 in the closed positions, in particular in a form-fit manner, e.g. by radially outwardly abutting portions (not shown) of the control member 39 or the like.

The control member 39 is axially moveable or shiftable in order to open the transportation lock 29. In particular, the control member 39 may be moved downwardly when completely closing the nebulizer 1 or its housing or completely pushing or snapping on the housing part 18. Preferably, the inner part 17 or ring 38 pushes the control member 39 downwardly or relatively to the gripping arms 35 so that the gripping arms 35 are released and, in particular, actively or positively opened or pivoted or flexed to open the transportation lock 29 and/or to release the container 3. In the shown embodiment, the control member 39 interacts with its axial end or an axial color or annular ring portion 40 with actuating portions 41 of the gripping arms 35 such that axially downward movement of the actuating portions 41 results in pivotation of the gripping arms 35 and radially outward flexing of the gripping arms 35. The flex characteristics of the gripping arms 35 depend on the used material, on the connection with member 37 and the like.

The control member 39 preferably opens the transportation lock 29 or gripping arms 35 positively.

Figs. 4 and 5 show the transportation lock 29 and the gripping arms 35 in the open position, i.e. wherein the container 3 is free to move axially. In particular, control member 39 is shown in its downward end position. In this position, the control member 39 is preferably locked or secured within the bottom part 18, in particular by force-fit or form-fit or by a snap-connection, so that the transportation lock 29 and the gripping arms 35 are held open permanently.

However, other constructional solutions of the transportation lock 29 are possible. In this regard, reference is made in particular to WO 2006/125577 A2 which shows some other constructional solutions, which can be realized as well.

Preferably, in the non-activated state, i.e. when the housing part 18 has not been pushed on fully, the nebulizer 1 may be locked to prevent tensioning of the pressure generator 5, i.e. in particular to prevent rotation of the inner part 17 relative to the upper housing part 16. This may be important when the nebulizer 1 is supplied in the delivery state with the pressure generator 5 not under tension. Accordingly, the inhaler 1 may have a barrier, so that the inner part 17 can only be rotated relative to the upper housing part 16 when the housing part 18 has been pushed fully on. Alternatively or additionally, the securing member 30 may block not only pushing on of the bottom part 18 in the delivery state, but also any rotation of the inner part 17 until the securing member 30 has been opened, released or removed.

Fig. 3 to 5 show the nebulizer 1 with a mouthpiece cover 42 covering the mouthpiece 13.

Generally, it should be pointed out that in the proposed nebulizer 1 the container 3 can preferably be inserted, i.e. incorporated in the nebulizer 1. Consequently, the container 3 is preferably a separate component. However, the container 3 may theoretically be formed directly by the nebulizer 1 or part of the nebulizer 1 or may otherwise be integrated in the nebulizer 1.

As already mentioned, individual features, aspects and / principles of the embodiments described may also be combined with one another as desired and may be used particularly in the known nebulizer according to Figs. 1 and 2 but also in similar or different nebulizers.

Unlike freestanding equipment or the like the proposed nebulizer 1 is preferably designed to be portable and in particular is a mobile hand operated device.

The proposed solution may, however, be used not only in the nebulizers 1 specifically described here but also in other nebulizers or inhalers, e.g. powder inhalers or so-called metered dose inhalers.

Preferably, the fluid 2 is a liquid, as already mentioned, especially an aqueous pharmaceutical formulation. However, it may also be some other pharmaceutical formulation, a suspension or the like.

According to an alternative embodiment the fluid 2 may also comprise particles or powder. In this case, instead of the expulsion nozzle 12, some other kind of supply device may be provided, especially an expulsion opening (not shown) or a supply channel (not shown) for supplying the fluid to or powder or the like into the mouthpiece 13. The optional air supply opening 15 then serves to supply ambient air preferably in parallel so as to general or allow an airflow with a sufficient volume for breathing in or inhaling through the mouthpiece 13.

If necessary the fluid 2 may also be atomized by means of a propellant gas.

Preferred ingredients and / or formulations of the preferably medicinal fluid 2 are listed in particular in WO 2009/047173 A2. As already stated, these may be aqueous or non-aqueous solutions, mixtures, formulations containing ethanol or free from solvent, or the like.

**List of reference numerals**

| | | | |
|---|---|---|---|
| 1 | nebulizer | 22 | piercing element |
| 2 | fluid | 23 | monitoring device |
| 3 | container | 24 | fluid outlet |
| 4 | bag | 25 | first closure |
| 5 | pressure generator | 26 | second closure |
| 6 | holder | 27 | closure part |
| 7 | drive spring | 28 | flange |
| 8 | locking element | 29 | transportation lock |
| 9 | conveying tube | 30 | securing member |
| 10 | non-return valve | 31 | latching lug |
| 11 | pressure chamber | 32 | latching recess |
| 12 | nozzle | 33 | latching arm |
| 13 | mouthpiece | 34 | vent opening |
| 14 | aerosol | 35 | gripping arm |
| 15 | air supply opening | 36 | edge |
| 16 | upper housing part | 37 | member |
| 17 | inner part | 38 | ring |
| 17a | upper part of the inner part | 39 | control member |
| 17b | lower part of the inner part | 40 | ring portion |
| 18 | housing part (lower part) | 41 | actuating portion |
| 19 | retaining element | 42 | mouthpiece cover |
| 20 | spring | | |
| 21 | container base | | |

## Claims

1. Nebulizer (1) for a fluid (2) comprising an insertable container (3) containing the fluid (2), the container (3) comprising a fluid outlet (24) for the fluid (2), the fluid outlet (24) being closed by a first closure (25) in a delivery state of the nebulizer (1) the first closure (25) being formed or supported by a closure part (27) extending from an outlet or head end of the container (3) into the container (3), wherein the container (3) is already disposed in the nebulizer (1) in the delivery state, the nebulizer (1) comprising a conveying element, which is a conveying tube (9), for conveying the fluid (2) from the container (3) to a pressure generator (5) of the nebulizer (1), the nebulizer (1) being constructed so that the first closure (25) and, thus, the fluid outlet (24) are opened inside the nebulizer (1) before or during first use of the nebulizer (1) by insertion of the conveying element,
**characterized in**
**that** the conveying tube (9) is already partly inserted into the container (3) in the delivery state, wherein the conveying tube (9) extends into the closure part (27) without opening the first closure (25), and
in that the nebulizer (1) comprises a transportation lock (29) for holding the container (3) unmovable in the delivery state.

2. Nebulizer according to claim 1, **characterized in that** the container (3) comprises a second closure (26) for closing the fluid outlet (24), wherein the second closure (26) is already opened by the conveying element in the delivery state.

3. Nebulizer according to claim 2, **characterized in that** the first and second closure (25, 26) are opened by means of the conveying element, particularly by piercing or insertion.

4. Nebulizer according to claim 2 or 3, **characterized in that** the second closure (26) is a seal of foil.

5. Nebulizer according to one of the preceding claims, **characterized in that** the nebulizer (1) comprises a housing which is closed only partly in the delivery state and completely closed for using the nebulizer (1), in particular wherein the first closure (26) is opened automatically when completely closing the housing.

6. Nebulizer according to one of the preceding claims, **characterized in that** the first closure (26) can be opened by moving the container (3) relative to the conveying element in longitudinal or axial direction.

7. Nebulizer according to claim 6, **characterized in that** the first closure (26) can be opened by telescopically pushing in the nebulizer (1), preferably a housing or housing part (18) of the nebulizer (1).

8. Nebulizer according to one of the preceding claims, **characterized in that** the first closure (25) is located inside the container (3).

9. Nebulizer according to one of claims 1-7, **characterized in that** the first closure (25) is spaced from an end of the container (3).

10. Nebulizer according to one of the preceding claims, **characterized in that** the container (3) is moveable preferably in a stroke action during conveying of the fluid, pressure generation and / or nebulization.

11. Nebulizer according to one of the preceding claims, **characterized in that** the transportation lock (29) can be released, opened or unlocked before or during opening of the first closure (25).

12. Nebulizer according to one of the preceding claims, **characterized in that** the transportation lock (29) can be released, opened or unlocked when completely closing a housing of the nebulizer (1).

13. Nebulizer according to one of the preceding claims, **characterized in that** the transportation lock (36) comprises at least one gripping arm (35) flexible to unlock or release the container (3).

14. Nebulizer according to one of the preceding claims, **characterized in that** the transportation lock (36) comprises a control member (39), particularly a ring, which is axially moveable, to unlock or release the container (3).

15. Nebulizer according to one of the preceding claims, **characterized in that** the nebulizer (1) is constructed as an inhaler, particularly for medical aerosol treatment.

## Patentansprüche

1. Zerstäuber (1) für ein Fluid (2), umfassend einen einschiebbaren Behälter (3), der das Fluid (2) enthält, wobei der Behälter (3) einen Fluidauslass (24) für das Fluid (2) umfasst, wobei der Fluidauslass (24) in einem Auslieferungszustand des Zerstäubers (1) durch einen ersten Verschluss (25) verschlossen ist, wobei der erste Verschluss (25) durch einen Verschlussteil (27) gebildet oder gehalten wird, der sich von einem Auslass- oder Kopfende des Behälters (3) in den Behälter (3) erstreckt, wobei der Behälter (3) im Auslieferungszustand bereits in dem Zerstäuber (1) angeordnet ist, wobei der Zerstäuber (1) ein Förderelement umfasst, bei dem es sich um ein Förderrohr (9) handelt, um das Fluid (2) von dem Behälter (3) zu einem Druckgenerator (5) des Zerstäubers (1) zu befördern, wobei der Zerstäuber (1) derart ausgebildet ist, dass der erste Verschluss (25) und somit der Fluidauslass (24) im Inneren des Zerstäubers (1) vor oder während der ersten Verwendung des Zerstäubers (1) geöffnet werden, indem das Förderelement eingeschoben wird,
**dadurch gekennzeichnet,**
**dass** das Förderrohr (9) im Auslieferungszustand bereits teilweise in den Behälter (3) eingeschoben ist, wobei sich das Förderrohr (9) in den Verschlussteil (27) erstreckt, ohne den ersten Verschluss (25) zu öffnen, und
**dass** der Zerstäuber (1) eine Transportverriegelung (29) umfasst, um den Behälter (3) im Auslieferungszustand unbeweglich zu halten.

2. Zerstäuber nach Anspruch 1, **dadurch gekennzeichnet, dass** der Behälter (3) einen zweiten Verschluss (26) zum Verschließen des Fluidauslasses (24) umfasst, wobei der zweite Verschluss (26) durch das Förderelement im Auslieferungszustand bereits geöffnet wurde.

3. Zerstäuber nach Anspruch 2, **dadurch gekennzeichnet, dass** der erste und zweite Verschluss (25, 26) mittels des Förderelements, insbesondere durch Durchstechen oder Einschieben, geöffnet werden.

4. Zerstäuber nach Anspruch 2 oder 3, **dadurch gekennzeichnet, dass** der zweite Verschluss (26) eine Versiegelung aus Folie ist.

5. Zerstäuber nach einem der vorhergehenden Ansprüche, **dadurch gekennzeichnet, dass** der Zerstäuber (1) ein Gehäuse umfasst, das im Auslieferungszustand nur teilweise geschlossen ist und zum Verwenden des Zerstäubers (1) vollständig geschlossen wird, wobei insbesondere der erste Verschluss (26) automatisch geöffnet wird, wenn das Gehäuse vollständig geschlossen wird.

6. Zerstäuber nach einem der vorhergehenden Ansprüche, **dadurch gekennzeichnet, dass** der erste Verschluss (26) geöffnet werden kann, indem der Behälter (3) relativ zu dem Förderelement in Längs- oder Achsrichtung bewegt wird.

7. Zerstäuber nach Anspruch 6, **dadurch gekennzeichnet, dass** der erste Verschluss (26) geöffnet werden kann, indem in dem Zerstäuber (1) vorzugsweise ein Gehäuse oder Gehäuseteil (18) des Zerstäubers (1) teleskopartig geschoben bzw. gedrückt wird.

8. Zerstäuber nach einem der vorhergehenden Ansprüche, **dadurch gekennzeichnet, dass** der erste Verschluss (25) im Inneren des Behälters (3) angeordnet ist.

9. Zerstäuber nach einem der Ansprüche 1 bis 7, **dadurch gekennzeichnet, dass** der erste Verschluss (25) von einem Ende des Behälters (3) beabstandet ist.

10. Zerstäuber nach einem der vorhergehenden Ansprüche, **dadurch gekennzeichnet, dass** der Behälter (3) vorzugsweise in einem Hubvorgang während der Förderung des Fluids, der Druckerzeugung und/oder der Zerstäubung bewegbar ist.

11. Zerstäuber nach einem der vorhergehenden Ansprüche, **dadurch gekennzeichnet, dass** die Transportverriegelung (29) vor oder während des Öffnens des ersten Verschlusses (25) freigegeben, geöffnet oder entriegelt werden kann.

12. Zerstäuber nach einem der vorhergehenden Ansprüche, **dadurch gekennzeichnet, dass** die Transportverriegelung (29) freigegeben, geöffnet oder entriegelt werden kann, wenn ein Gehäuse des Zerstäubers (1) vollständig geöffnet wird.

13. Zerstäuber nach einem der vorhergehenden Ansprüche, **dadurch gekennzeichnet, dass** die Transportverriegelung (36) wenigstens einen Greifarm (35) umfasst, der biegsam ist, um den Behälter (3) zu entriegeln oder freizugeben.

14. Zerstäuber nach einem der vorhergehenden Ansprüche, **dadurch gekennzeichnet, dass** die Transportverriegelung (36) ein Steuerungselement (39), insbesondere einen Ring, umfasst, der axial beweglich ist, um den Behälter (3) zu entriegeln oder freizugeben.

15. Zerstäuber nach einem der vorhergehenden Ansprüche, **dadurch gekennzeichnet, dass** der Zerstäuber (1) als Inhalator, insbesondere zur medizinischen Aerosolbehandlung, ausgebildet ist.

## Revendications

1. Nébuliseur (1) pour un fluide (2) comprenant un récipient (3) insérable contenant le fluide (2), le récipient (3) comprenant une sortie de fluide (24) pour le fluide (2), la sortie de fluide (24) étant fermée par une première fermeture (25) dans un état de distribution du nébuliseur (1), la première fermeture (25) étant formée ou supportée par une partie de fermeture (27) s'étendant d'une sortie ou extrémité de tête du récipient (3) dans le récipient (3), dans lequel le récipient (3) est déjà disposé dans le nébuliseur (1) dans l'état de distribution, le nébuliseur (1) comprenant un élément de transport, qui est un tube de transport (9), pour le transport du fluide (2) du récipient (3) à un générateur de pression (5) du nébuliseur (1), le nébuliseur (1) étant construit de sorte que la première fermeture (25) et, ainsi, la sortie de fluide (24) sont ouvertes à l'intérieur du nébuliseur (1) avant ou pendant la première utilisation du nébuliseur (1) par insertion de l'élément de transport,
**caractérisé en ce**
**que** le tube de transport (9) est déjà partiellement inséré dans le récipient (3) dans l'état de distribution, dans lequel le tube de transport (9) s'étend dans la partie de fermeture (27) sans ouverture de la première fermeture (25), et
en ce que le nébuliseur (1) comprend un verrou de transport (29) pour maintenir le récipient (3) immobile dans l'état de distribution.

2. Nébuliseur selon la revendication 1, **caractérisé en ce que** le récipient (3) comprend une deuxième fermeture (26) pour la fermeture de la sortie de fluide (24), dans lequel la deuxième fermeture (26) est déjà ouverte par l'élément de transport dans l'état de distribution.

3. Nébuliseur selon la revendication 2, **caractérisé en ce que** les première et deuxième fermetures (25, 26) sont ouvertes au moyen de l'élément de transport, en particulier par perçage ou insertion.

4. Nébuliseur selon la revendication 2 ou 3, **caractérisé en ce que** la deuxième fermeture (26) est un joint de feuille.

5. Nébuliseur selon l'une des revendications précédentes, **caractérisé en ce que** le nébuliseur (1) comprend un boîtier qui est fermé seulement partiellement dans l'état de distribution et complètement fermé pour l'utilisation du nébuliseur (1), en particulier dans lequel la première fermeture (26) est ouverte automatiquement lors de la fermeture complète du boîtier.

6. Nébuliseur selon l'une des revendications précédentes, **caractérisé en ce que** la première fermeture (26) peut être ouverte par déplacement du récipient (3) par rapport à l'élément de transport dans la direction longitudinale ou axiale.

7. Nébuliseur selon la revendication 6, **caractérisé en ce que** la première fermeture (26) peut être ouverte en poussant télescopiquement dans le nébuliseur (1), de préférence un boîtier ou partie de boîtier (18) du nébuliseur (1).

8. Nébuliseur selon l'une des revendications précédentes, **caractérisé en ce que** la première fermeture (25) est située à l'intérieur du récipient (3).

9. Nébuliseur selon l'une des revendications 1 à 7, **caractérisé en ce que** la première fermeture (25) est espacée d'une extrémité du récipient (3).

10. Nébuliseur selon l'une des revendications précédentes, **caractérisé en ce que** le récipient (3) est de préférence mobile dans une action de course pendant le transport du fluide, la génération de pression et/ou la nébulisation.

11. Nébuliseur selon l'une des revendications précédentes, **caractérisé en ce que** le verrou de transport (29) peut être libéré, ouvert ou déverrouillé avant ou pendant l'ouverture de la première fermeture (25).

12. Nébuliseur selon l'une des revendications précédentes, **caractérisé en ce que** le verrou de transport (29) peut être libéré, ouvert ou déverrouillé lors de la fermeture complète d'un boîtier du nébuliseur (1).

13. Nébuliseur selon l'une des revendications précédentes, **caractérisé en ce que** le verrou de transport (36) comprend au moins un bras de préhension (35) souple pour déverrouiller ou libérer le récipient (3).

14. Nébuliseur selon l'une des revendications précédentes, **caractérisé en ce que** le verrou de transport (36) comprend un élément de commande (39), en particulier une bague, qui est axialement mobile, pour déverrouiller ou libérer le récipient (3).

15. Nébuliseur selon l'une des revendications précédentes, **caractérisé en ce que** le nébuliseur (1) est construit en tant qu'inhalateur, en particulier pour un traitement aérosol médical.
